# EUROPÄISCHE PATENTSCHRIFT

(11) **EP 1 051 962 B1**
(45) Veröffentlichungstag und Bekanntmachung des Hinweises auf die Patenterteilung: **25.07.2007**
(21) Anmeldenummer: 00810373.1
(22) Anmeldetag: 03.05.2000
(51) Int. Cl.: A61K 8/22, A61K 8/25, A61Q 11/00

(54) **Zahnpflegemittel enthaltend Perlit**
Dental care compositions containing perlite
Compositions de soins dentaires contenant de la perlite

(30) Priorität: 06.05.1999 EP 99810396
(43) Veröffentlichungstag der Anmeldung: 15.11.2000
(73) Patentinhaber: KerrHawe SA, 6934 Bioggio (CH)
(72) Erfinder: Kilcher, Beat, 6935 Bosco Luganese (CH); Balmelli, Patrizia, 6932 Breganzona (CH); Silber, Gert, Dr., 6946 Ponte Capriasca (CH); Von Weissenfluh, Beat A., 6925 Gentilino (CH)
(74) Vertreter: AMMANN PATENTANWÄLTE AG BERN

(56) Entgegenhaltungen:
- EP-A- 0 528 756
- WO-A-94/15577
- WO-A-96/09033
- WO-A-96/09034
- DE-A- 3 639 844
- INCI by CTFA Washington, 7th Edition, 1997, pp. 928-932.

## Beschreibung

Die vorliegende Erfindung betrifft ein Zahnpflegemittel zur vorbeugenden Zahnhygiene gemäss Oberbegriff des Anspruchs 1.

Ein derartiges Zahnpflegemittel besteht in der Regel aus drei verschiedenen, nach Härte eingeordneten Pasten (hart, mittel, weich), deren Struktur nicht mit einer üblicherweise allgemein verwendeten Zahnpasta vergleichbar ist. Sie entspricht eher einer fast krümeligen Polierpaste. Diese krümelige Struktur ist bei Anwendung in der Mundhöhle mit einem Poliergerät notwendig, um Substanzverlust durch z.B. Spritzen zu vermeiden. Die Pasten werden in Ergänzung zur täglichen Zahnpflege von Zahnhygienikern benutzt, um hartnäckig anhaftende Zahnbeläge zu entfernen. Diese zusätzliche Behandlung soll die Zahnpflege derart unterstützen, dass Karies verringert und somit zahnärztliche Behandlung weitgehend überflüssig wird.

Eine derartige wasserhaltige Paste, die Fällungskieselsäure als hauptsächliche Putzkörperkomponente, daneben aber auch Perlit, enthält, ist in der EP-A-0 268 763 beschrieben. Eine andere Variante, die zwingend hydrophobe pyrogene Kieselsäure zur Stabilisierung des Perlits enthält, ist in EP-A-528 756 beschrieben.

In der Anwendung zeigen diese Zahnpflegemittel, auch Prophylaxepasten genannt, verschiedene Probleme:

Die zum Zerbröseln neigende Konsistenz erschwert das Entnehmen aus Vorrats- oder Portionenbehältern und das Aufbringen auf die Zahnreinigungswerkzeuge (Pastenträger) der Zahnhygieniker.

Aus dem gleichen Grund ist es auch nicht möglich, die bekannten Prophylaxepasten in die bevorzugten Portionen für jeweils eine Anwendung problemlos zu portionieren.

Schliesslich zeigen die bekannten Prophylaxepasten beim Kontakt mit der Saliva des Patienten die bekannte Neigung, sich spontan mit dieser zu vermischen und vom Pastenträger heruntergeschleudert zu werden. Diese Werkzeuge zeichnen sich z.B. durch einen Drehzahlbereich von 2000 bis 6000 Umdrehungen pro Minute aus. Es wird jedoch oft empfohlen, keine Drehzahlen oberhalb 3000 Umdrehungen pro Minute einzusetzen, da ansonsten die Paste vom Werkzeug heruntergeschleudert wird.

Eine Aufgabe der vorliegenden Erfindung besteht daher darin, ein Zahnpflegemittel für die vorbeugende Zahnhygiene anzugeben, das verbesserte Anwendungseigenschaften aufweist.

Ein derartiges Zahnpflegemittel ist im Anspruch 1 angegeben. Die weiteren Ansprüche geben bevorzugte Ausführungsformen und Anwendungen an.

Die erfindungsgemässe Paste zeichnet sich demgemäss dadurch aus, dass sie einen Putzkörper, der im wesentlichen aus scharfkantigen, plättchenförmigen Partikeln eines Gesteins, bevorzugt Perlit, besteht, und einen Anteil an Polyether enthält.

Der Polyether, im weiteren nichtionisches Tensid genannt, übernimmt zugleich die Funktion eines Lösungsmittels, sein Anteil und seine Art stellen also die Fliesseigenschaften des Zahnpflegemittels ein. Als besonders geeignet hat sich hierfür der unter dem INN Macrogol bekannte Polyethylenglykol erwiesen. Es wird vermutet, dass das nichtionische Tensid eine Art Hülle wenigstens um die Gesteinspartikel bildet, wobei diese Hülle zugleich als Bindungsvermittler zu den übrigen, freien Molekülen des Tensides wirkt. Aus dieser Überlegung heraus ist es verständlich, dass insbesondere mässig langkettige Tenside besonders gute Wirksamkeit aufweisen. Versuche haben aber auch gezeigt, dass zu langkettige Spezies dazu führen, dass die Paste ihre Geschmeidigkeit verliert und zu trocken oder zu bröselig wird. Es hat sich auch gezeigt, dass in der Regel ein Zusatz eines Emulgators oder eines Emulgatorgemischs erforderlich ist, insbesondere wenn auch bei sorgfältiger Auswahl des Polyethers keine genügende Geschmeidigkeit erreicht wird.

Plättchenförmige Putzkörperpartikel neigen dazu, sich in der Anwendung derart auszurichten, dass Riefenbildung und andere unerwünschte Folgen der Einwirkung der Prophylaxepaste gegenüber z.B. eher kugeligen, scharfkantigen Partikeln deutlich verringert werden. Aus diesem Befund heraus kann erwartet werden, dass auch andere mineralische Materialien, also Gesteine, die zu Partikeln geeigneter Grösse und Form zerkleinerbar sind, ähnlich dem Prototypen Perlit verwendbar sind wie z. B. Bentonit, Vermiculit, Zeolithe, Bimsstein. Die Plättchen können dabei auch gekrümmt sein, so wie diejenigen von Perlit schalenförmig sind ähnlich Eierschalenfragmenten. Bemerkenswert ist auch hinsichtlich Perlit, dass die Partikel bei der Anwendung zerbrechen, dabei aber scharfkantig bleiben und damit ihre Reinigungswirkung bewahren, wenn auch zunehmend schonender werden entsprechend einem Übergehen von grobem zu feinerem Schleifmaterial.

Weitere Details der Verwendung von Perlit und anderen Gesteinsarten in Prophylaxepasten sind der EP-A-528 756 zu entnehmen.

Die Erfindung soll weiter an einem Ausführungsbeispiel erläutert werden. Prozentangaben sind, wie überall in der Beschreibung und den Ansprüchen, Gewichtsprozente.

Eine erfindungsgemässe Prophylaxepaste weist folgende obligatorische Komponenten auf:
- Perlit (expandiert) 10 - 80 Gew.-%, bevorzugt 35 - 55 Gew.-%
- Macrogol 20 - 80 Gew.-%, bevorzugt 40 - 50 Gew.-%
- Emulgator oder -gemisch 0 - 20 Gew.-%, bevorzugt 1 - 10 Gew.-%
- Zusatz- und Wirkstoffe 0 - 20 Gew.-%, bevorzugt 0 - 10 Gew.-%
- Füllstoffe 0 - 70 Gew.-%.

Das Macrogol ist dabei jeweils ein käufliches Produkt mit bestimmtem Molekulargewicht. Es übernimmt als eine Hauptkomponente die Funktionen der üblicherweise vorhandenen Flüssigkeit wie Wasser, und sein Anteil und seine Art beeinflussen die Rheologie, insbesondere Viskosität und Geschmeidigkeit. Die Paste ist mithin im wesentlichen frei von Wasser und anderen niedermolekularen, bei Zimmertemperatur (298 K) flüssigen und insbesondere als Lösungsmittel verwendbaren Komponenten wie den in dieser Anwendung bekannten Stoffen Ethanol, Propylenglykol, Glycerin. Als niedermolekular könnte also ein Molekulargewicht von höchstens 100 angesehen werden. Molekulargewichte von Macrogol von 200 - 1000, bevorzugt 200 - 600, haben sich als besonders geeignet erwiesen. Es können die marktgängigen Produkte mit eng spezifiziertem, mittlerem oder eindeutigem Molekulargewicht pur oder in Mischung verwendet werden. Es ist zu erwarten, dass durch Mischen von Tensiden verschiedenen Molekulargewichts eine feinere Einstellung der Anwendungseigenschaften, insbesondere der Rheologie, möglich ist. Bei einem zunehmenden Anteil an höhermolekulargewichtigem Macrogol ist z.B. eine Erhöhung der Viskosität zu erwarten.

Das Perlit weist einen mittleren Partikeldurchmesser von ca. 30 µm auf, wobei sich 99 % im Grössenbereich von 1 µm bis 200 µm Durchmesser befinden.

Als Emulgator bzw. Komponenten des Emulgatorgemisches können dienen:

Höhere Fettalkohole, insbesondere mit 8 bis 20 Kohlenstoffatomen in der Kette, z.B. Cetylalkohol, Laurylalkohol, Stearylalkohol und/oder Fettsäureester eines Polyoxyethylens, z.B. ethoxyliertes Rizinusöl (Eumulgin R040, Henkel KG a.A., Deutschland) oder Polyoxyethylenstearat.

Zusätzlich kann die Prophylaxepaste noch die üblichen Zusatz- und Wirkstoffe in den nötigen Anteilen, bevorzugt von insgesamt 0 % bis 10 %, aufweisen:

Antiseptikum, Fluoridsalz, Aromen, Süssstoff, Konservierungsmittel (Antioxidanz, Antimikrobiotikum) und evtl. zusätzliche, sekundäre Abrasivmaterialien. Die genannten sekundären Abrasivkörper stellen zusammen mit dem Perlit, oder allgemein Gestein, den Putzkörper dar.

Zur Feineinstellung der Abrasivität können der Paste Füllstoffe zugesetzt werden. Anforderungen an die Füllstoffe sind, dass sie im wesentlichen keinen eigenen Beitrag zur Abrasivität leisten, also diese nicht erhöhen, und auch die Konsistenz der Paste nicht nachteilig beeinflussen. Derartige Füllstoffe sind dem Fachmann aus dem allgemeinen Fachwissen zugänglich. Beispielsweise können als Füllstoff höhermolekulare Polyethylenglykole und Wachsalkohole dienen. Die höhermolekularen Polyethylenglykole können ebenfalls die als Macrogol benannten sein, wobei als Füllstoffe Produkte mit höherem Molekulargewicht als diejenigen zur Anwendung kommen, die als nichtionische Tenside dienen.

In der Praxis wurde gefunden, dass die so herstellbare Prophylaxepaste sehr gut in die gewünschten Einmal-Dosen portionierbar ist, insgesamt eine eher cremige Konsistenz im Vergleich mit den bekannten Pasten aufweist und trotzdem weniger dazu neigt, bei Kontakt mit Saliva vom Werkzeug heruntergeschleudert zu werden. Bemerkenswert ist, dass die Paste gegenüber Wasseraufnahme tolerant ist, d.h. ihre Anwendungseigenschaften in feuchter Umgebung nur so wenig ändert bzw. sie hinreichend lange bewahrt, dass sie wirksam bleibt und insbesondere das Verspritzen in wesentlich geringerem Ausmass als bei bekannten Pasten auftritt.

Ein weiterer Vorteil ist die gefühlsmässig angenehme, cremige Konsistenz.

Eine beispielhafte Zusammensetzung ist:

**Beispiel 1**

| Komponente | Anteil [Gew.-%] |
|---|---|
| Aromastoff | 2 % |
| Cetylalkohol | 3 % |
| Emulgator (Eumulgin RO40 [Henkel, Deutschland]) | 3 % |
| Farbstoff (rotes Eisenoxid) | 1,7 % |
| Natriumflorid | 0,3 % |
| Perlit | 45 % |
| Polyethylenglycol 400 [Macrogol] | 43 % |
| Süsstoff (Aspartam (Warenzeichen)) | 1 % |
| Titandioxid [Pigment] | 2 % |

Ausgehend von der Beschreibung des Ausführungsbeispieles sind dem Fachmann Varianten im Bereich der Erfindung zugänglich. Denkbar ist es, modifizierte Polyethylenglykole oder allgemein Polyalkylenglykole einzusetzen, z.B. mit entsprechenden Substituenten an der Molekülkette oder den Enden, verzweigte Arten, veresterte oder veretherte Formen, usw. Weiter verallgemeinert kann auch an Polyalkylether oder generell Polyether gedacht werden. Denkbar sind auch andere Strukturelemente der Polymerkette wie z.B. Propylen oder Methylen sowohl als Homopolymer wie auch als Copolymer. Bevorzugt sind allgemein Arten mit 1 bis 4 Kohlenstoffatomen pro Alkylenglykol-Einheit.

Bei der Wahl des Gesteins und dessen Partikelgrössen ist ein grosser Spielraum für die Anpassung an den Einsatzzweck vorhersehbar.

Denkbar ist auch der Zusatz von niedermolekularen Lösungsmitteln (Wasser, Propylenglykol etc.) in geringen Mengen, bevorzugt in einem Gesamtanteil von höchstens 10 Gew.-%, weiter bevorzugt höchstens 5 Gew.-%, um eine Optimierung der Konsistenz durchzuführen.

## Patentansprüche

1. Zahnpflegemittel für die vorbeugende Zahnhygiene,
**dadurch gekennzeichnet,**
**dass** es plättchenförmige, ebene oder gekrümmte Partikel eines Gesteins, insbesondere von Perlit, als hauptsächlichen Bestandteil des Putzkörpers und einen Anteil von 20 - 80 Gew.-% an Polyether aufweist, Putzkörper und Polyether zusammen mindestens 30 Gew.-% des Zahnpflegemittels darstellen und 0 - 20 Gew.-% Emulgator vorhanden sind, wobei wenigstens der Polyetheranteil zur Einstellung der Fliesseigenschaften dient.

2. Zahnpflegemittel gemäss Anspruch 1, **dadurch gekennzeichnet, dass** es höchstens 10 Gew.-%, bevorzugt höchstens 5 Gew.-% Wasser und andere niedermolekulare, insbesondere ein Molekulargewicht von höchstens 100 aufweisende, bei 298 K flüssige Substanzen wie Alkohole enthält und insbesondere bevorzugt im wesentlichen frei von diesen Komponenten ist.

3. Zahnpflegemittel gemäss Anspruch 1 oder 2,
**dadurch gekennzeichnet,**
**dass** es 10 - 80 Gew.-%, bevorzugt 35 - 55 Gew.-% Perlit aufweist.

4. Zahnpflegemittel gemäss einem der Ansprüche 1 bis 3, **dadurch gekennzeichnet, dass** der Polyether ein Polyalkylether ist.

5. Zahnpflegemittel gemäss einem der Ansprüche 1 bis 4, **dadurch gekennzeichnet, dass** der Polyether ein modifizierter oder unmodifizierter Polyalkylenglykol ist, bevorzugt mit Alkylenglykolgruppen mit 1 bis 4 Kohlenstoffatomen und weiter bevorzugt mit im wesentlichen ausschliesslich Ethylenglykol-Gruppen.

6. Zahnpflegemittel gemäss einem der Ansprüche 1 bis 5, **dadurch gekennzeichnet, dass** es 20 - 80 Gew.-%, bevorzugt 40
- 50 Gew.-% Macrogol (Polyethylenglykol) als Polyether aufweist.

7. Zahnpflegemittel gemäss einem der Ansprüche 1 bis 6,
**dadurch gekennzeichnet,**
**dass** sich der Polyether aus Molekülen mit einem Molekulargewicht im wesentlichen im Bereich von 200 bis 1000, bevorzugt 200 bis 600, zusammensetzt.

8. Zahnpflegemittel gemäss einem der Ansprüche 1 bis 7,
**dadurch gekennzeichnet,**
**dass** zusätzlich mindestens eine der folgenden Komponenten vorhanden ist:
- Antiseptikum
- Fluoridsalz
- Aromastoff
- Süssstoff
- Konservierungsmittel, insbesondere Antibiotikum, Antioxidanz
und/oder
- mindestens ein sekundärer Abrasivstoff im Putzkörper.

9. Zahnpflegemittel gemäss Anspruch 8, **dadurch gekennzeichnet, dass** der Gesamtanteil der zusätzlichen Komponenten maximal 20 Gew.-% und bevorzugt 0 - 10 Gew.-% ist.

10. Zahnpflegemittel gemäss einem der Ansprüche 1 bis 9,
**dadurch gekennzeichnet,**
**dass** der Emulgatoranteil 1 - 10 Gew.-% beträgt.

11. Zahnpflegemittel gemäss einem der Ansprüche 1 bis 10,
**dadurch gekennzeichnet,**
**dass** der Emulgatoranteil eine oder mehrere folgender Substanzen bzw. Substanzklassen umfasst:
- höherer Fettalkohol, insbesondere mit 8 bis 20 Kohlenstoffatomen in der Kohlenstoffkette, bevorzugt ausgewählt aus Cetylalkohol, Stearylalkohol, Laurylalkohol,
- Fettsäureester von Polyoxyalkylethern, bevorzugt ausgewählt aus ethoxyliertem Rizinusöl, Polyoxyethylenstearat.

12. Zahnpflegemittel gemäss einem der Ansprüche 1 bis 11, **dadurch gekennzeichnet, dass** es 0 bis 70 Gew.-% an Füllstoffen enthält, um die Abrasivität des Zahnpflegemittel einzustellen, wobei die Füllstoffe so gewählt sind, dass sie weder die Konsistenz nachteilig beeinflussen noch eine eigene Abrasiwirkung aufweisen.

13. Zahnpflegemittel gemäss einem der Ansprüche 1 bis 12, **dadurch gekennzeichnet, dass** der Putzkörper und der Anteil an Polyether zusammen mindestens 70 Gew.-% des Zahnpflegemittels ausmachen.

## Claims

1. Dental care composition for prophylactic dental hygiene, **characterized in that** it contains plate-shaped, flat or curved rock particles, more particularly of perlite, as the principal constituent of the cleaning body and a content of 20 to 80 % by weight of a polyether, that the cleaning body and the polyether together represent at least 30 % by weight of the dental care composition, and that 0 to 20 % by weight of an emulsifier are provided, at least the polyether fraction serving for the adjustment of the flow properties.

2. Dental care composition according to claim 1, **characterised in that** it contains at most 10 % by weight, preferably at most 5 % by weight of water and other low molecular substances that have a molecular weight of no more than 100 and are liquid at 298 K, such as alcohols, and more preferably that it is substantially free of such components.

3. Dental care composition according to claim 1 or 2, **characterised in that** it comprises 10 to 80 % by weight, preferably 35 to 55 % by weight of perlite.

4. Dental care composition according to one of claims 1 to 3, **characterised in that** the polyether is a polyalkyl ether.

5. Dental care composition according to one of claims 1 to 4, **characterised in that** the polyether is a modified or unmodified polyalkylene glycol, preferably one with alkylene glycol groups having from 1 to 4 carbon atoms and more preferably essentially with ethylene glycol groups only.

6. Dental care composition according to one of claims 1 to 5, **characterised in that** it comprises 20 to 80 % by weight, preferably 40 to 50 % by weight of Macrogol (polyethylene glycol) as the polyether.

7. Dental care composition according to one of claims 1 to 6, **characterised in that** the polyether is composed of molecules whose molecular weight is essentially comprised within the range of 200 to 1,000, preferably 200 to 600.

8. Dental care composition according to one of claims 1 to 7, **characterised in that** at least one of the following components is additionally contained:
- antiseptic
- fluoride salt
- flavouring agent
- sweetening agent
- preservatives, particularly an antibiotic, antioxidant, and/or
- at least one secondary abrasive material in the cleaning body.

9. Dental care composition according to claim 8, **characterised in that** the total content of the additional components at most 20 % by weight and preferably 0 to 10 % by weight.

10. Dental care composition according to one of claims 1 to 9, **characterised in that** the emulsifier content is 1 to 10 % by weight.

11. Dental care composition according to one of claims 1 to 10, **characterised in that** the emulsifier fraction comprises one or several ones of the following substances or substance classes:
- higher fatty alcohol, more particularly one whose carbon chain has 8 to 20 carbon atoms, preferably selected from cetyl alcohol, stearyl alcohol, lauryl alcohol,
- fatty acid esters of polyoxyalkyl ethers, preferably selected from ethoxylated castor oil, polyoxyethylene stearate.

12. Dental care composition according to one of claims 1 to 11, **characterised in that** it contains from 0 to 70 % by weight of fillers for adjusting the abrasiveness of the dental care composition, the fillers being selected such that they do not affect the consistency nor exhibit an abrasive effect themselves.

13. Dental care composition according to one of claims 1 to 12, **characterised in that** the cleaning body and the polyether fraction together account for at least 70 % by weight of the dental care composition.

## Revendications

1. Composition de soins dentaires pour l'hygiène dentaire prophylactique, **caractérisée en ce qu'**elle comprend des particules en forme de plaquettes planes ou courbées d'une matière rocheuse, plus particulièrement de perlite, comme composant principal du corps de nettoyage et une teneur de 20 à 80 % en poids en polyéther, que le corps de nettoyage et le polyéther représentent conjointement au moins 30 % en poids de la composition de soins dentaires et que 0 à 20 % en poids d'un émulsifiant sont contenus, du moins la fraction du polyéther servant à ajuster les propriétés rhéologiques.

2. Composition de soins dentaires selon la revendication 1, **caractérisée en ce qu'**elle contient 10 % en poids au plus, préférablement 5 % en poids au plus, d'eau et d'autres substances à poids moléculaire bas ayant plus particulièrement un poids moléculaire de 100 au plus et qui sont liquides à 298 K, tels que des alcools, et en particulier qu'elle est essentiellement libre de ces composants.

3. Composition de soins dentaires selon la revendication 1 ou 2, **caractérisée en ce qu'**elle comprend 10 à 80 % en poids, préférablement 35 à 55 % en poids de perlite.

4. Composition de soins dentaires selon l'une des revendications 1 à 3, **caractérisée en ce que** le polyéther est un éther de polyalkyle.

5. Composition de soins dentaires selon l'une des revendications 1 à 4, **caractérisée en ce que** le polyéther est un polyalkylèneglycol modifié ou non modifié qui contient préférablement des groupes alkylèneglycol ayant 1 à 4 atomes de carbone et plus préférablement essentiellement des groupes éthylèneglycol uniquement.

6. Composition de soins dentaires selon l'une des revendications 1 à 5, **caractérisée en ce qu'**elle comprend 20 à 80 % en poids, préférablement 40 à 50 % en poids de macrogol (polyéthylèneglycol) en tant que polyéther.

7. Composition de soins dentaires selon l'une des revendications 1 à 6, **caractérisée en ce** le polyéther est composé de molécules dont le poids moléculaire est essentiellement compris dans la plage de 200 à 1000, préférablement de 200 à 600.

8. Composition de soins dentaires selon l'une des revendications 1 à 7, **caractérisée en ce que** l'un au moins des composants suivants est encore contenu:
- antiseptique
- sel de fluorure
- aromatisant
- édulcorant
- agents conservateurs, plus particulièrement un antibiotique, un antioxydant, et/ou
- au moins une matière abrasive secondaire dans le corps de nettoyage.

9. Composition de soins dentaires selon la revendication 8, **caractérisée en ce** la teneur totale en composants additionnels est égale à 20 % en poids au maximum et préférablement à 0 à 10 % en poids.

10. Composition de soins dentaires selon l'une des revendications 1 à 9, **caractérisée en ce que** la teneur en émulsifiant est de 1 à 10 % en poids.

11. Composition de soins dentaires selon l'une des revendications 1 à 10, **caractérisée en ce que** la fraction de l'émulsifiant comprend l'une ou plusieurs des substances ou classes de substances suivantes:
- alcool gras supérieur, plus particulièrement dont la chaîne des carbones comprend 8 à 20 atomes de carbone, préférablement choisi parmi l'alcool cétylique, l'alcool stéarique, l'alcool laurique,
- esters d'acides gras de polyoxyalkyle-éthers, préférablement choisis parmi l'huile de ricin éthoxylée, le stéarate de polyoxyéthylène.

12. Composition de soins dentaires selon l'une des revendications 1 à 11, **caractérisée en ce qu'**elle contient 0 à 70 % en poids de matières de charge afin d'ajuster l'abrasivité de la composition de soins dentaires, les matières de charge étant choisies de telle manière qu'elles n'affectent pas la consistance et ne présentent elles-mêmes aucun effet abrasif.

13. Composition de soins dentaires selon l'une des revendications 1 à 12, **caractérisée en ce que** le corps de nettoyage et la fraction du polyéther représentent conjointement au moins 70 % en poids de la composition de soins dentaires.
